# DEMANDE DE BREVET EUROPEEN

(11) **EP 3 821 874 A1**
(43) Date de publication de la demande: **19.05.2021**
(21) Numéro de dépôt: 20020511.0
(22) Date de dépôt: 06.11.2020
(51) Int. Cl.: A61K 8/02, A61K 8/27, A61K 8/36, A61K 8/44, A61K 8/9789, A61K 8/9794, A61Q 1/02, A61Q 1/06, A61Q 1/10, A61Q 3/02

(54) **COMPOSITION COSMÉTIQUE POUR LE MAQUILLAGE AVEC EXTRAIT COLORANT D'ORIGINE VÉGÉTALE ET COMPOSÉ SOLIDE LAMELLAIRE INSOLUBLE DANS L'EAU ET NON SILICIÉ**

(30) Priorité: 14.11.2019 FR 1912741
(71) Demandeur: Le Rouge Français, 92140 Clamart (FR)
(72) Inventeur: CARPENTIER, Elodie, 92140 Clamart (FR); GHEZAILI, Salem, 92140 Clamart (FR)

(57) **Abrégé**

Composition cosmétique pour le maquillage de la peau ou des lèvres ou des cils ou des ongles comprenant un ou plusieurs extraits colorants d'origine végétale (a) et un ou plusieurs composés solides lamellaires insolubles dans l'eau et non siliciés (b), le ou les composés (b) étant choisis de préférence parmi la guanine,l'oxyde de zinc et les stéarates de métaux divalents ou leurs mélanges. Procédé d'application. Utilisation en particulier dans le domaine des rouges à lèvres.

## Description

Dans le domaine du maquillage il est connu d'utiliser des pigments colorés d'origine minérale ou d'origine organique synthétique soit seuls soit en association, dispersés dans des formules pulvérulentes ou dans des formules liquides, crèmes ou solides, contenant notamment des corps gras.

Les pigments cités ci dessus et notamment les pigments organiques synthétiques peuvent cependant poser des problèmes d'innocuité. Par ailleurs ces pigments ne sont pas issus de sources renouvelables et leur empreinte carbone n'est pas favorable.

Il existe donc un besoin de rechercher des substituts à ces pigments organiques synthétiques.

Il a donc été proposé d'utiliser des pigments colorés organiques d'origine naturelle animale comme le carmin de cochenille largement utilisé dans les produits cosmétiques de maquillage. mais l'origine animale de ce produit peut aussi rebuter l'utilisateur.

Des pigments colorés d'origine végétale ont donc été proposés pour se substituer aux pigments colorés existant comme l'extrait de garance (Rubia Tinctorum extract) figurant dans des produits commerciaux. Les demandes de brevets chinoises CN109260036 de GONG CHENGFENG et CN106667874 de PENG ZHICHENG proposent aussi respectivement l'utilisation entre autres d'anthocyanes, de lutéine, de pigment rouge de tomate d'une part et d'autre part du pigment de fruit rouge du dragon (pitaya).

Mais le plus souvent ces pigments colorés d'origine végétale sont moins performants que les pigments minéraux ou organiques synthétiques en particulier dans le domaine de la couvrance du produit cosmétique sur son substrat d'application.

Il existe donc un besoin de mettre à disposition des compositions améliorées pour le maquillage et en particulier pour le maquillage de la peau ou des lèvres ou des cils ou des ongles, notamment au niveau de la couverture du substrat anatomique.

A cet effet, la demanderesse préconise des compositions pour le maquillage de la peau ou des lèvres ou des cils ou des ongles comprenant un ou plusieurs extraits colorants d'origine végétale (a) et un ou plusieurs composés solides lamellaires insolubles dans l'eau et non siliciés (b).

Par composition pour le maquillage, on entend au sens de la présente invention une composition apportant de la couleur au substrat anatomique (peau ou lèvres ou cils ou ongles) sur lequel elle est appliquée.

Par composé insoluble dans l'eau, on entend au sens de la présente invention un composé dont la solubilité dans l'eau est inférieure à 0,25% (0,25 g pour 100 ml), de préférence inférieure à 0,05%, mieux inférieure à 0,005% à température ambiante (plage de 15°C à 37°C) et à pression atmosphérique (760 mm de mercure).

Par composé lamellaire on entend au sens de la présente invention que le composé à température ambiante et à pression atmosphérique présente un arrangement des cristaux en feuillets ou plaquettes.

Par composé solide on entend qu'a température ambiante et pression atmosphérique le composé (b) se présente sous forme d'un solide. Les solides (b) de l'invention sont de préférence non colorés c'est à dire blancs ou transparents.

Par composé non silicié on entend au sens de la présente invention un composé ne comprenant pas d'atome de silicium dans sa formule chimique.

Le ou les composés (b) peuvent être d'origine minérale ou organique.

A titre de composés minéraux présentant les caractéristiques des composés (b) on peut citer l'oxyde de zinc.

A titre de composés organiques présentant ces caractéristiques on peut citer la guanine et les stéarates de métaux divalents et notamment les stéarates de zinc ou de magnésium et préférentiellement le stéarate de zinc.

Les compositions de l'invention présentent alors une couvrance améliorée. Par couvrance améliorée, on entend une meilleure couverture du substrat nécessitant une moindre quantité de composition pour obtenir l'effet colorant souhaité. Cette moindre quantité entraîne une moindre épaisseur de produit et un meilleur toucher.

Parmi les documents de l'art antérieur évoquant les composés(b) on peut citer le document JP2005220099 de PIGEON CORP envisageant l'association dans un rouge à lèvres avec corps gras de pigments et d'oxyde de zinc plus oxyde de titane, oxyde de cérium et filtre UV mais il s'agit de pigments inorganiques et le but était d'obtenir une composition émolliente avec protection UV pour application sur des enfants sans effet irritant.

De même les documents de KAO CORP JP2016117653 et JP 2017025049 évoquent le maquillage de la peau, respectivement sous forme de poudres ou d'émulsions, avec des associations oxyde de zinc et pigments, essentiellement pour un meilleur toucher ou état de la peau, sans mention des pigments végétaux.

Le document JPH02178209 de PACIFIC CHEM CO évoque quant à lui des gels cosmétiques contenant des cristaux liquides, des agents perlescents dont la guanine éventuellement associés à des pigments organiques ou minéraux, pour améliorer l'apparence et l'hydratation. Ces gels peuvent être utilisés pour le maquillage des yeux. Les extraits colorants d'origine végétale ne sont pas envisagés.

Dans le document CN107823062 de XUZHOU SHANGQIN ELECTRONICS AND TECH CO LTD est décrite l'association de pigments et de stéarate de zinc dans le domaine des rouges pour la peau et les lèvres pour un effet santé naturel mais il n'y a pas mention de pigments d'origine végétale. Le seul extrait mentionné est celui de Riz gluant qui n'est pas un extrait de plantes colorant.

Enfin le document JPH02270813 de KAO CORP cite des poudres cosmétiques utilisées pour le maquillage à effet plus durable pouvant contenir du stéarate de magnésium et des pigments de type organique synthétique ou minéral. Les extraits colorants d'origine végétale ne sont pas évoqués.

Les compositions de l'invention comprennent un ou plusieurs extraits colorants d'origine végétale (a).

Par extrait colorant d'origine végétale on entend au sens de la présente invention un extrait contenant un ou plusieurs colorants issus d'une plante ou d'une sécrétion de plante.

Les extraits colorants d'origine végétale (a) peuvent provenir de tous types de végétaux appartenant au règne végétal. Ils peuvent ainsi provenir de cryptogammes mais plus préférentiellement de phanérogames. Ils peuvent en particulier provenir d'arbres, d'arbustes, de plantes vertes, de plantes à fleurs, de cactées, de graminées.

Ils peuvent provenir de toutes les parties de la plante et en particulier des racines, des tiges ou des troncs, des feuilles, des fleurs et des fruits. Ils peuvent aussi être issus de sécrétions ou exsudats de plantes produits par les dites plantes, en particulier en réaction ou en prévention à une agression.

Ils peuvent être obtenus par broyage, par extraction à l'eau ou aux solvants ou au dioxyde de carbone supercritique, par attaque chimique ou enzymatique ou par une combinaison de ces moyens. Il peut y avoir après une opération de concentration en actifs colorants par filtration ou par évaporation de solvant. En final une addition d'adjuvants est possible pour faciliter la mise en œuvre.

Ces extraits (a) peuvent être solubles ou insolubles dans l'eau à température ambiante et à pression atmosphérique.

A titre d'extraits colorants d'origine végétale (a) on peut notamment citer de manière non limitative ceux issus de:
Garance (exemple d'espèce Rubia Tinctorum)
Tomate (exemple d'espèce Solanum Lycopersicum)
Quebracho(exemple d'espèce Schinopsis quebracho-colorado)
Pernambouc (exemple d'espèce Caesalpinia Echinata)
Châtaignier (exemple d'espèce Castanea Sativa)
Curcuma (exemple d'espèce Curcuma Tumeric)
Sorgho (exermple d'espèce Sorghum Bicolor)
Cachou (exemple d'espèce Acacia Catechu)
Rocouyer (exemple d'espèce Bixa Orellana)
Sappan (exemple d'espèce Caesalpinia Sappan)
Genipa (exemple d'espèce Genipa Americana)
Indigotier (exemple d'espèce Indigofera Tinctoria)
Chou rouge (exemple d'espèce Brassica Oleracea)
Réséda (exemple d'espèce Reseda Luteola)
Sumac (exemple d'espèce Rhus Semialata)

De préférence les extraits colorants d'origine végétale (a) sont choisis parmi ceux issus de la garance, de la tomate, du sorgho et du châtaignier, du sumac.

Les extraits colorants d'origine végétale de l'invention sont de préférence dispersés dans la composition cosmétique les contenant. Par dispersé on entend que l' extrait est insolubilisé ou pas complètement solubilisé dans la composition à température ambiante et à pression atmosphérique.

La concentration en extraits colorants d'origine végétale (a) dans les compositions de l'invention varie de préférence de 0,1% à 60% en poids , plus préférentiellement de 1 à 50%, mieux de 2 à 40%, encore mieux de 4 à 35% en poids par rapport au poids total de la composition.

Il est possible d'ajouter aux extraits colorants d'origine végétale (a) d'autres colorants solubles dans l'eau ou de préférence insolubles dans l'eau à température ambiante et à pression atmosphérique et en particulier des pigments organiques de synthèse ou des pigments minéraux.

De préférence les extraits colorants d'origine végétale (a) sont les seuls colorants présents dans les compositions de l'invention.

Dans une première variante de l'invention les compositions de l'invention contiennent de l'oxyde de zinc à titre de composé (b).

Cet oxyde de zinc peut être d'origine naturelle (wurzite) ou synthétique. Sa solubilité dans l'eau à 30°C et à pression atmosphérique est de 0,00016 g%.

De préférence l'oxyde de zinc utilisé dans l'invention est non hydrophobisé par un traitement chimique de surface.

Dans une seconde variante de l'invention les compositions de l'invention contiennent un stéarate de métal divalent à titre de composé (b).

De préférence le métal divalent est choisi parmi le zinc ou le magnésium. Encore mieux le métal divalent est le zinc. Le stéarate peut être d'origine végétale, animale et synthétique. Quand il est d'origine végétale, il est le plus souvent issu d'huile de palme mais peut être issu d'autres huiles végétales. La solubilité dans l'eau du stéarate de zinc à 20°Cet à pression atmosphérique est de 0,00009 g %. Celle du stéarate de magnésium est de 0,0004 g%.

Dans une troisième variante de l'invention les compositions de l'invention contiennent de la guanine à titre de composé (b).

Cette guanine peut être d'origine naturelle ou synthétique. Sa solubilité dans l'eau à 37°C est de 0,21 g%.

Lorsque la guanine est d'origine naturelle, elle peut être isolée à partir d'excréments d'oiseaux. On peut aussi l'obtenir à partir d'écailles de poissons.

Dans une quatrième variante de l'invention, à titre de composés (b), il est bien entendu possible d'associer oxyde de zinc, stéarate de métal divalent, de préférence de zinc, et guanine dans les compositions de l'invention, soit les trois ensemble ou deux par deux. L'association préférée est celle d'oxyde zinc et de stéarate de zinc.

De préférence le composé (b) est choisi parmi les composés à propriétés lubrifiantes et en particulier parmi les agents lubrifiants comportant du zinc dans leur structure comme l'oxyde de zinc et le stéarate de zinc. Par propriétés lubrifiantes on entend l'aptitude à diminuer les frottements entre les composés en mouvement et en particulier entre les particules.

La concentration en composés (b) des compositions de l'invention varie de préférence de 0,1% à 40% en poids, plus préférentiellement de 1,5 à 35%, mieux de 2,5 à 30%, encore mieux de 6 à 25% en poids par rapport au poids total de la composition.

Les compositions de l'invention peuvent être aqueuses ou anhydres.

Par anhydre on entend au sens de l'invention une composition contenant à température ambiante moins de 5% d'eau en poids, encore mieux moins de 1% d'eau en poids et préférentiellement ne contenant pas d'eau, en particulier d'eau ajoutée.

Par aqueuse on entend au sens de l'invention une composition contenant à température ambiante de 5% à 99,01% d'eau en poids. De préférence si elle est aqueuse, la composition comprend à température ambiante de 25 à 98% d'eau , mieux de 40 à 95% d'eau en poids.

De préférence la ou les compositions de l'invention sont anhydres.

Les compositions de l'invention contiennent de préférence un ou plusieurs corps gras.

Le ou les corps gras sont choisis parmi les huiles ou les cires.

Les compositions de l'invention peuvent contenir une ou plusieurs huiles.

Par huile on entend au sens de la présente invention un composé non miscible à l'eau liquide à température ambiante et à pression atmosphérique.

Les huiles peuvent être polaires ou apolaires, volatiles ou non volatiles (une huile est dite non volatile quand sa pression de vapeur est inférieure à 0,02 mm de mercure à pression atmosphérique et à température ambiante).

Les huiles peuvent être hydrocarbonées, d'origine végétale, minérale ou synthétique. Elles peuvent être aussi siliconées ou fluorées.

Les huiles siliconées peuvent être des polydiméthylsiloxanes linéaires ou ramifiés ou cycliques porteurs éventuellement de groupes aryle et en particulier phényle. Les huiles hydrocarbonées apolaires sont choisies de préférence parmi l'huile de paraffine, les isoparaffines (isododécane, isodécanes), les alcanes linéaires (n-dodécane), le squalane, l'eicosane, les polybutylènes ou les polyisobutylènes hydrogénés ou non, les polydècènes hydrogénés ou non.

Les huiles polaires sont de préférence hydrocarbonées. Elles peuvent être choisies parmi les alcools gras ramifiés ou insaturés comme l'alcool oléique et l'octyldodécanol. Elles sont de préférence choisies parmi les triglycérides d'origine végétale ou synthétique et les esters d'acides gras autres que les triglycérides.

Parmi les huiles d'origine végétale on peut notamment citer l'huile d'amande douce, l'huile d'argan, l'huile d'avocat, l'huile d'arachide, l'huile de camélia, l'huile de carthame, l'huile de calophyllum, l'huile de colza, l'huile de coprah, l'huile de coriandre, l'huile de courge, l'huile de germes de blé, l'huile de jojoba ou cire liquide de jojoba, l'huile de lin, l'huile de macadamia, l'huile de germes de maïs, l'huile de noisette, l'huile de noix, l'huile de vernonia, l'huile de noyaux d'abricot, l'huile d'olive, l'huile d'onagre, l'huile de palme, l'huile de passiflore, l'huile de pépins de raisin, l'huile de ricin, l'huile de rosier, l'huile de seigle, l'huile de sésame, l'huile de son de riz, l'huile de soja et l'huile de tournesol.

De préférence l'huile ou les huiles est ou sont choisies parmi les huiles végétales.

Les compositions de l'invention peuvent contenir une ou plusieurs cires. Par cire on entend au sens de la présente invention un composé lipophile solide à température ambiante et à pression atmosphérique, à changement d'état solide/liquide réversible, ayant un point de fusion supérieur à 30°C et de préférence inférieur à 120°C. Le point de fusion de la cire peut être mesuré à l'aide d'un calorimètre à balayage différentiel (DSC), par exemple celui proposé par la société METTLER sous la référence DSC 30.

Les cires peuvent être hydrocarbonées, siliconées ou fluorées et être d'origine naturelle minérale, animale, végétale ou d'origine synthétique. Elles peuvent être polaires ou apolaires.

Comme cires apolaires on peut citer les cires microcristallines, les cires de paraffines, l'ozokérite, les cires de polyéthylènes.

Les cires polaires sont de préférence hydrocarbonées et encore plus préférentiellement choisies parmi les cires esters comprenant au moins une fonction ester et les cires alcools comprenant au moins une fonction alcool.

Les cires esters sont de préférence choisies parmi les esters d'acide(s) gras et/ou d'alcools gras sous forme solide à température ambiante et à pression atmosphérique.

Ces cires esters peuvent être issues d'huiles animales ou végétales hydrogénées.

Les cires alcools ont de préférence choisies parmi les alcools gras sous forme solide à température ambiante et à pression atmosphérique tels que l'alcool cétylique ou l'alcool cétylstéarylique.

Les cires d'origine naturelle sont de préférence choisies parmi la cire d'abeille, la cire de carnauba, la cire de candellila, la cire de son de riz, la cire d'ouricury, la cire d'alfa, la cire de fibres de liège, la cire de canne à sucre, la cire du Japon, la cire de sumac, la cire de montan, la cire d'orange, la cire de laurier, la cire de pomme, le beurre de karité. De préférence la ou les cires est ou sont choisies parmi les cires végétales. Mieux la ou les compositions ne contiennent que des cires végétales. Plus préférentiellement la ou les cires est ou sont choisies parmi la cire de carnauba, la cire de candellila et le beurre de karité.

De préférence les compositions de l'invention contiennent au moins une huile végétale et au moins une cire végétale.

Dans les compositions de l'invention la teneur en corps gras peut varier de 1 à 98% en poids, mieux de 10 à 95% en poids, encore mieux de 20 à 95% en poids par rapport au poids total de la composition.

Les compositions selon l'invention peuvent comprendre un ou plusieurs agents tensioactifs choisis parmi les agents tensioactifs anioniques, non-ioniques, cationiques ou amphotères.

Les compositions selon l'invention peuvent en outre comprendre un ou plusieurs additifs classiques bien connus dans la technique, différents des composés définis précédemment.

A titre d'exemples d'additifs utilisables selon l'invention, on peut citer les polymères associatifs ou non associatifs, ioniques ou non-ioniques et en particulier les polymères épaississants, les épaississants minéraux, les filtres UVA et/ou UVB, les agents adoucissants, les solvants, les agents hydratants, les vitamines, les pro-vitamines, les acides aminés, les parfums, les agents peptisants, les agents conservateurs, les agents antioxydants, les agents alcalinisants ou acidifiants (notamment pour réguler le pH des compositions aqueuses ou pour salifier les ingrédients), les agents fixateurs de couleur, les agents anti-rides.

Les compositions de l'invention sont destinées au maquillage de la peau des lèvres, des cils ou des ongles afin de modifier la couleur de ces substrats.

Elles peuvent être sous forme de poudres, de solides compacts, de solutions ou d'émulsions.

De préférence les compositions de l'invention sont destinées au maquillage de la peau ou des lèvres.

De préférence les compositions de l'invention sont sous forme de poudres ou de solides compacts.

Les compositions pour le maquillage de la peau peuvent être des fonds de teints, des ombres ou fards à paupières.

Encore plus préférentiellement, les compositions de l'invention sont destinées au maquillage des lèvres.

Mieux la composition de l'invention est une composition solide pour le maquillage des lèvres.

Encore mieux la composition de l'invention est un bâton de rouge à lèvres.

L'invention couvre également un procédé de maquillage de la peau ou des lèvres ou des cils ou des ongles, de préférence de la peau ou des lèvres , et encore plus préférentiellement des lèvres consistant à appliquer sur le substrat anatomique (peau ou lèvres ou cils ou ongles) une composition telle que décrite ci dessus sans rinçage subséquent.

### [Exemples]

Les exemples ci-dessous illustrent l'invention sans toutefois en limiter la portée.

### Exemple 1

La composition décrite dans le tableau 1 ci dessous est celle d'un bâton de rouge à lèvres.

Après application sur les lèvres, il permet d'obtenir sur celles-ci une belle coloration.

La couvrance obtenue est très bonne.

**[Tableaux 1]**

| Ingrédient | Quantité en g pour 100g de composition |
|---|---|
| Extrait de de racines de garance (extrait colorant d'origine végétale (a)) | 6 |
| Oxyde de zinc (composé (b)) | 14,4 |
| Stéarate de zinc (composé (b)) | 1,6 |
| Huile de ricin | 19,25 |
| Huile de tournesol | 32,27 |
| Cire de Carnauba | 18 |
| Beurre de karité | 5,6 |
| Alun de potassium | 1,83 |
| Carbonate de sodium | 0,87 |
| Tocopherol | 0,18 |

### Exemple 2

Cet exemple ne diffère de l'exemple 1 que par le remplacement des 16 g de l'association oxyde de zinc + stéarate de zinc par 16 g d'oxyde de zinc. Les résultats obtenus sont similaires.

### Exemple 3

Cet exemple ne diffère de l'exemple 1 que par le remplacement des 16 g de l'association oxyde de zinc + stéarate de zinc par 16 g de stéarate de zinc. Les résultats obtenus sont similaires.

### Exemple 4

Cet exemple ne diffère de l'exemple 1 que par le remplacement des 16 g de l'association oxyde de zinc + stéarate de zinc par 16 g de guanine. Les résultats obtenus sont similaires.

## Revendications

1. Composition cosmétique pour le maquillage de la peau ou des lèvres ou des cils ou des ongles comprenant un ou plusieurs extraits colorants d'origine végétale (a) et un ou plusieurs composés solides lamellaires insolubles dans l'eau et non siliciés (b).

2. Composition selon la revendication 1 dans laquelle le ou les composés (b) sont choisis parmi la guanine, l'oxyde de zinc, les stéarates de métaux divalents et leurs mélanges.

3. Composition selon les revendications 1 et 2 dans laquelle le ou les composés (b) sont des agents lubrifiants.

4. Composition selon l'une quelconque des revendications précédentes dans laquelle le ou les composés (b) sont choisis parmi l'oxyde de zinc, le stéarate de zinc et leurs mélanges.

5. Composition selon l'une quelconque des revendications précédentes dans laquelle la concentration en composés (b) varie de 0,1 à 40%, plus préférentiellement de 1,5 à 35%, mieux de 2,5 à 30%, encore mieux de 6 à 25% en poids par rapport au poids total de la composition.

6. Composition selon l'une quelconque des revendications précédentes dans laquelle le ou les extraits (a) sont issus des plantes suivantes : garance, tomate, quebracho, pernambouc, châtaignier, curcuma, sorgho, cachou, rocouyer, sappan, genipa, indigotier , chou rouge, réséda, sumac.

7. Composition selon l'une quelconque des revendications précédentes dans laquelle le ou les extraits (a) sont sous forme dispersée.

8. Composition selon l'une quelconque des revendications précédentes dans laquelle la concentration en extraits (a) varie de 0,1 à 60%, plus préférentiellement de 1 à 50%, mieux de 2 à 40%, encore mieux de 4 à 35% en poids par rapport au poids total de la composition.

9. Composition selon l'une quelconque des revendication précédentes destinée au maquillage de la peau ou des lèvres.

10. Composition selon l'une quelconque des revendications précédentes destinée au maquillage des lèvres, de préférence sous forme d'un bâton de rouge à lèvres.

11. Composition selon l'une quelconque des revendication précédentes **caractérisée par le fait qu'**elle est anhydre.

12. Procédé de maquillage de la peau ou des lèvres ou des cils ou des ongles, de préférence de la peau ou des lèvres, et encore plus préférentiellement des lèvres consistant à appliquer sur le substrat anatomique une composition selon l'une quelconque des revendications 1 à 11 sans rinçage subséquent.
